# EUROPÄISCHE PATENTSCHRIFT

(11) **EP 2 499 487 B1**
(45) Veröffentlichungstag und Bekanntmachung des Hinweises auf die Patenterteilung: **06.08.2014**
(21) Anmeldenummer: 10768195.9
(22) Anmeldetag: 24.09.2010
(51) Int. Cl.: G01N 33/50

(54) **VERFAHREN ZUR TESTUNG VERSCHIEDENER, AUSGEWÄHLTER WERKSTOFFE UND/ODER OBERFLÄCHENSTRUKTUREN FÜR DIE KULTUR VON ZELLEN**
PROCESS FOR TESTING DIFFERENT MATERIALS AND/OR SURFACE STRUCTURES FOR CELL CULTURE
PROCÉDÉ POUR EXAMINER DIFFÉRENTS MATÉRIAUX ET/OU STRUCTURES EXTÉRIEURES POUR LA CULTURE DE CELLULES

(30) Priorität: 09.11.2009 DE 102009046525
(43) Veröffentlichungstag der Anmeldung: 19.09.2012
(73) Patentinhaber: Helmholtz-Zentrum Geesthacht Zentrum für Material- und Küstenforschung GmbH, 21502 Geesthacht (DE); Charité - Universitätsmedizin Berlin, 10117 Berlin (DE)
(72) Erfinder: KLEIN, Frank, 14089 Berlin (DE); WEIGEL, Thomas, 14552 Michendorf (DE); KRATZ, Karl, 14615 Berlin (DE); JUNG, Friedrich, 01259 Dresden (DE); HIEBL, Bernhard, 06112 Halle an der Saale (DE); LENDLEIN, Andreas, 14167 Berlin (DE); LÜTZOW, Karola, 12167 Berlin (DE); KURTZ, Andreas, 10437 Berlin (DE); REINKE, Petra, 10117 Berlin (DE); RÖMHILD, Andy, 13503 Berlin (DE)
(74) Vertreter: Gulde & Partner
(86) Internationale Anmeldenummer: PCT/EP2010/064128
(87) Internationale Veröffentlichungsnummer: WO 2011/054601

(56) Entgegenhaltungen:
- WO-A2-03/083044
- WO-A2-2006/116752
- US-A1- 2008 160 539
- HIEBL B ET AL: "Cytocompatibility testing of cell culture modules fabricated from specific candidate biomaterials using injection molding", JOURNAL OF BIOTECHNOLOGY, ELSEVIER SCIENCE PUBLISHERS, AMSTERDAM, NL, Bd. 148, Nr. 1, 1. Juli 2010 (2010-07-01), Seiten 76-82, XP027096381, ISSN: 0168-1656 [gefunden am 2010-06-19]

## Beschreibung

In der Zellkultur spielt der Einfluss von Werkstoffen, die mit den zu kultivierenden Zellen in irgendeiner Form in Kontakt kommen, eine große Rolle. So erfolgt z.B. die Entwicklung und Anwendung neuer polymerer Werkstoffe in der Medizintechnik, Pharmazie und Biotechnologie durch die Testung auf Verträglichkeit mit pro- und/oder eukaryotischen Zellen i.d.R. durch Anzüchten (Kultivieren) der Zellen auf dem neuen Werkstoff. Dabei ist es wünschenswert ein System für die Zell- und/oder Gewebkultur bereitzustellen, dass es ermöglicht, ohne Beeinflussung durch Fremdmaterialien den Einfluß polymerbasierter Werkstoffe auf Zellen in direktem Kontakt reproduzierbar, kostengünstig und schnell zu untersuchen, wobei das System grundsätzlich für eine high throughput Anwendung geeignet sein soll.

Im Bereich der Zell- und Gewebekulturtechnik kommen sterile Einweg-Zellkultur-Träger zum Einsatz, sogenannte Multiwell-Platten. Diese Träger werden derzeit aus wenigen Werkstoffen, bevorzugt Polystrol (PS), hergestellt und angeboten. Die Multiwell-Platten sind dabei in unterschiedlichen Formen kommerziell verfügbar, die weitestgehend normiert sind. Die Zellkulturträger sind sterilisierbar, bevorzugt für die Mikroskopie geeignet und sollen gewährleisten, dass reproduzierbare Untersuchungen durchgeführt werden können. Es werden beispielsweise in WO 2006/116752 A2 Vorrichtungen aus biokompatiblem Material für die Zellkultur sowie Verfahren zur Herstellung derselben offenbart, während in WO 03/083044 A2 becherförmige Einsätze für Mikrotiterplatten offenbart werden, die passgenau in die Vertiefungen der Mikrotiterplatten eingesetzt werden können.

Durch sich ergebende neue Anwendungsfelder im Bereich der Medizin, Biologie oder Biotechnologie (z.B. Zelltherapie, Tissue engineering, biotechnologische Produktionstechniken) sind die Erfordernisse hinsichtlich neuer Werkstoffe gestiegen. Zur Überprüfung vollkommen neuer Werkstoffe, inklusive auch der Modifikation von Werkstoffen hinsichtlich chemisch oder physikalisch bedingter Parameter, wie beispielsweise der Oberflächenbeschaffenheit, wird der Werkstoff in einen Zellkulturträger fixiert und über einen definierten Zeitraum mit Zellen in flüssigen Zellkulturmedien inkubiert. Um die Werkstoffe in dem Zellkulturträger stabil fixieren zu können, werden die Werkstoffe i.d.R. mit Fixierhilfen gestützt und beschwert, die aus einem Werkstoff gefertigt sind, welches sich von dem zu testenden Werkstoff und/oder dem Werkstoff des verwendeten Zellkulturträgers unterscheidet (u.a. Agarose, Glas, Teflon, Edelstahl, Polyester). Das Testergebnis ist damit das Resultat einer Beeinflussung der Zellen durch mehrere unterschiedliche Werkstoffe und kann nicht mehr einem bestimmten Werkstoff allein zugeschrieben werden. Des Weiteren kann mit den Fixierhilfen nicht sicher ausgeschlossen werden, dass es während der Testung zu einer Formveränderung der Werkstoffe kommt. So kann sich beispielsweise die planare Struktur von Folien hin zu einer gewellten Struktur verändern. Daraus kann eine wesentliche Veränderung der mikro- und nanostrukturierten Topographie des Werkstoffes resultieren, die auf die Interaktion der Zellen mit dem Werkstoff erheblichen Einfluss nehmen kann.

Aufgabe der vorliegenden Erfindung ist es einen oder mehrere Nachteile des Standes der Technik zu überwinden oder abzumildern. Insbesondere ist es eine Aufgabe der Erfindung ein Verfahren bereitzustellen, welches die parallele Testung von verschiedenen Werkstoffen und Oberflächen bzgl. ihrer Eignung für die Kultur von Zellen erlaubt.

Die vorliegende Erfindung stellt ein Verfahren bereit zur Testung verschiedener, ausgewählter Werkstoffe und/oder Oberflächenstrukturen für die Kultur von Zellen und/oder Mikroorganismen, umfassend die Schritte:
i) Bereitstellung einer Mehrzahl von zu testenden verschiedenen, ausgewählten Werkstoffen und/oder Oberflächenstrukturen in Form von becherförmigen Einsätzen, wobei die becherförmigen Einsätze derart dimensioniert sind, dass ein becherförmiger Einsatz jeweils in eine Vertiefung einer für die Zellkultur geeigneten Multiwell-Platte im Wesentlichen passgenau einsetzbar ist, wobei jeder becherförmige Einsatz Wände, einen Boden und auf der dem Boden abgewandten Seite eine Öffnung aufweist, wobei Wände und Boden der becherförmigen Einsätze flüssigkeitsdicht ausgestaltet sind und wobei jeder becherförmige Einsatz mindestens auf der Bodeninnenseite jeweils einen ausgewählten Werkstoff und/oder eine ausgewählte Oberflächenstruktur aufweist;
ii) Einsetzen der becherförmigen Einsätze in Vertiefungen von Multiwell-Platten entsprechender Dimensionierung, so dass die Außenseite des Becherbodens in Richtung der Innenseite des Bodens einer Vertiefung der Multiwell-Platte zeigt;
iii) Kultur der Zellen im Lumen der becherförmigen Einsätze.

Die Verwendung eines becherförmigen Einsatzes ermöglicht eine Testung der Zelleffekte und/oder Kultivierung der Zellen bezüglich eines Werkstoffs und/oder einer Oberflächenstruktur ohne Beeinflussung durch Fremdmaterialien auf der Basis konventioneller Multiwell-Platten. Die erfindungsgemäße Lösung des Problems besteht darin, einen becherförmigen Einsatz bereitzustellen und einzusetzen, der in seinen Abmessungen so beschaffen ist, dass er in die Vertiefung (engl. "well") einer handelsüblichen, standardisierten Multiwell-Platte passt. Dabei weist der becherförmige Einsatz mindestens auf der Bodeninnenseite einen ausgewählten Werkstoff und/oder eine ausgewählte Oberflächenstruktur auf. Die Geometrie des becherförmigen Einsatzes kann dabei den Erfordernissen der Zellen sowohl in x-y-Richtung (z.B. Realisierung einer kreisförmigen, sternförmigen oder andersförmigen Grundfläche des Inserts) wie auch in z-Richtung angepasst werden. Dabei beschreiben die x- und y-Richtungen die Ausdehnung des Einsatzes in einer flächigen Ebene parallel zu der Ebene in der die Öffnungen der Vertiefungen der Multiwell-Platten angeordnet sind, während die z-Richtung die Richtung der Tiefenausdehnung der Vertiefungen der Multiwell-Platte beschreibt. Dieser becherförmige Einsatz bietet eine genau definierte Oberfläche, die sowohl in chemisch wie auch physikalisch modifizierter Form angeboten werden kann. Dadurch ist sichergestellt, dass die Zellen während der Kultur auschließlich gegenüber nur einem, dem zu untersuchenden Werkstoff exponiert sind. Der becherförmige Einsatz kann während der Kultur in der Multiwell-Platte nicht aufschwimmen, da die Kultivierung der Zellen im Lumen des becherförmigen Einsatzes erfolgt. Der becherförmige Einsatz kann problemlos in ein anderes Zellkulturträger-System überführt werden, da er entnehmbar ist. Da der becherförmige Einsatz an die Abmessungen handelsüblicher, standardisierter Multiwell-Platten angepaßt ist, kann die in einem Labor üblicherweise bereits vorhandene Ausstattung zur Zellkultivierung ohne Einschränkungen weiter benutzt werden. Die Eigenschaften der Sterilisierbarkeit, Mikroskopierbarkeit und Reproduzierbarkeit sind bei solch einem System ebenfalls gegeben. Die Verwendung unterschiedlicher Materialien und/oder Oberflächenstrukturen in Form von becherförmigen Einsätzen für konventionelle Multiwell-Platten erlaubt den Einsatz standardisierter Testverfahren oder -systeme und ist daher zeit- und kostensparender als die aufwendige Fixierung von Materialproben mit Hilfsmitteln in Vertiefungen von Multiwell-Platten. Die Verwendung unterschiedlicher Werkstoffe und/oder Oberflächenstrukturen in Form von becherförmigen Einsätzen für konventionelle Multiwell-Platten erlaubt einen automatisierten Einsatz im Rahmen von Anwendungen mit High-Throughput-Charakter. Durch das einfache Überführen der becherförmigen Einsätze aus einer Vertiefung einer ersten Multiwell-Platte in eine andere Vertiefung ggf. einer anderen zweiten Multiwell-Platte, kann der Ablauf der Testung flexibel erweitert und/oder geändert werden. Dabei kann innerhalb einer Multiwell-Platte von vergleichbaren Zellkulturbedingungen ausgegangen werden. Dies reduziert den systematischen Fehler der Testansätze.

Typischerweise stehen am Anfang der Entwicklung neuer Polymersysteme meist nur kleine Mengen des neuen Werkstoffs zur Verfügung. Da die benötigte Menge zur Herstellung von becherförmigen Einsätzen kleiner ist, als die benötigte Menge zur Herstellung einer ganzen Multiwell-Platte, können basierend auf der vorliegenden Erfindung auch Kleinstmengen an neuen Werkstoffen effektiv genutzt bzw. einer Testung zugeführt werden.

Bei dem erfindungsgemäßen Verfahren handelt es sich um ein Verfahren zur Testung von verschiedenen, ausgewählten Werkstoffen und/oder Oberflächenstrukturen für die Kultur von Zellen und/oder Mikroorganismen. Dabei werden unter dem Begriff "Zellen" sowohl einzelne Zellen, als auch eine Mehrzahl von Zellen eines Typs oder Mischungen von Zellen unterschiedlichen Typs sowie Zellverbände und Gewebe- und/oder Organteile verstanden. Dabei können die Zellen primären Ursprungs sein oder es kann sich um Zelllinien, immortalisierte Zellen oder jede Art von Mischungen daraus handeln. Die Zellen können eukaryotische Zellen umfassen oder daraus bestehen, sie können aber auch prokaryotische Zellen umfassen oder daraus bestehen. Unter Mikroorganismen werden hier einzellige Lebewesen verstanden, insbesondere Bakterien, Pilze wie z.B. Hefen und Algen.

Bei den verschiedenen, ausgewählten Werkstoffen handelt es sich um Werkstoffe, die in verarbeiteter Form im Wesentlichen nicht wasserlöslich sind und die unter üblichen Zellkulturbedingungen, beispielsweise in einem Temperaturbereich von 0°C bis 60°C, als Feststoff vorliegen. Bevorzugt handelt es sich dabei um polymere Werkstoffe, z.B. um Homopolymere oder Copolymere, wobei die polymeren Werkstoffe nach der Polymerisation weiteren Behandlungen und/oder Modifikationen unterzogen werden können.

Bei den verschiedenen, ausgewählten Oberflächenstrukturen handelt es sich um Oberflächen von Feststoffen, die sich beispielsweise unterscheiden können bzgl. der Werkstoffzusammensetzung, der dreidimensionalen Form, des Oberflächenpotentials, der Porosität, der Mikro- und/oder Makrostrukturierung, der Hydrophobizität, der Hydrophilizität und/oder der Ausstattung mit funktionellen Gruppen. Bei den Feststoffen kann es sich dabei sowohl um bekannte, feste Werkstoffe als auch um neue, feste Werkstoffe handeln. Die ausgewählten Oberflächenstrukturen können beispielsweise durch Modifikation von bereits bestehenden Oberflächen hergestellt sein.

Im erfindungsgemäßen Verfahren werden die zu testenden Werkstoffe und/oder Oberflächenstrukturen in Form von becherförmigen Einsätzen bereit gestellt. Dabei sind die becherförmigen Einsätze derart dimensioniert, dass jeweils ein becherförmiger Einsatz in eine Vertiefung (oder "well") einer für die Zellkultur geeigneten Multiwell-Platte im Wesentlichen passgenau einsetzbar ist. Unter einer für die Zellkultur geeigneten Multiwell-Platte werden solche, ggf. handelsüblichen, standardisierten Zellkulturträger verstanden, die auf einem einzigen Träger mehrere unabhängige Kompartimente zur Aufnahme und Kultur von Zellen aufweisen. Üblicherweise weist eine Multiwell-Platte eine Basis auf, die eine Mehrzahl von separaten Vertiefungen für die Kultur von Zellen umfasst und einen Deckel, der i.d.R. derart ausgestaltet ist, dass der Deckel alle Vertiefungen einer Multiwell-Platte bedecken kann. Üblicherweise sind diese Multiwell-Platten aus einem Polymer gefertigt, insbesondere aus Polystyrol. Bevorzugt werden Multiwell-Platten im 4-well, 6-well, 12-well, 24-well, 48-well, 96-well oder 384-well Format eingesetzt, bevorzugt wird eine 24-well Multiwell-Platte verwendet. Die Vertiefungen der Multiwell-Platten können einen Boden aufweisen, der entweder planar ausgestaltet ist oder eine Krümmung aufweist, bevorzugt werden Multiwell-Platten verwendet deren Vertiefungen einen planaren Boden aufweisen. Geeignete Multiwell-Platten werden von einer Reihe von kommerziellen Herstellen angeboten und vertrieben, beispielsweise von Corning Inc., BD Biosciences, Biochrom AG, Greiner GmbH, Nunc GmbH & Co.KG.

Im erfindungsgemäßen Verfahren kann mindestens ein Teil der becherförmigen Einsätze derart dimensioniert sein, dass ein becherförmiger Einsatz im Wesentlichen passgenau in eine Vertiefung einer Multiwell-Platte im 4-well, 6-well, 12-well, 24-well, 48-well, 96-well oder 384-well Format einsetzbar ist, bevorzugt in eine Vertiefung einer 24-well Multiwell-Platte.

Die becherförmigen Einsätze sind derart ausgestaltet und dimensioniert, dass ein becherförmiger Einsatz im Wesentlichen passgenau in eine Vertiefung einer Multiwell-Platte einsetzbar ist. Dabei wird unter einer passgenauen Einsetzbarkeit verstanden, dass der becherförmige Einsatz derart in die Vertiefung einsetzbar ist, dass der becherförmige Einsatz in der Vertiefung nicht soviel Spiel hat, dass der becherförmige Einsatz bei moderatem Schütteln umkippen könnte. Insbesondere ist der becherförmige Einsatz derart dimensioniert, dass der becherförmige Einsatz in der Vertiefung aufrecht anordenbar ist. Bevorzugt beträgt der Spalt zwischen der Außenwand des becherförmigen Einsatzes und einer Innenoberfläche einer Seitenwand der Vertiefung an seiner schmalsten Stelle nicht mehr als 5 mm, bevorzugt nicht mehr als 1 mm, ganz besonders bevorzugt berühren sich die Außenwand des becherförmigen Einsatzes und eine Innenoberfläche einer Seitenwand der Vertiefung in einem Bereich, wobei dieser Bereich eine radiale Ausdehnung aufweisen kann. Die tatsächliche Ausgestaltung und Dimensionierung der becherförmigen Einsätze wird durch die Ausgestaltung und Dimensionierung der Vertiefungen der zu verwendenden Multiwell-Platte bestimmt.

Der becherförmige Einsatz weist eine Öffnung, Becherwände und einen Boden auf, wobei die Wände mit dem Boden das Lumen des betreffenden Einsatzes definieren, welches durch die Öffnung zugänglich ist. Dabei sind Wände und Boden der becherförmigen Einsätze flüssigkeitsdicht ausgestaltet, so dass ein Lumen entsteht, welchem lediglich über die Öffnung Flüssigkeit zugeführt und/oder abgeführt werden kann. Die Stärke der Wände und/oder des Bodens eines becherförmigen Einsatzes ist dabei derart gewählt, dass der becherförmige Einsatz einerseits genügend Stabilität aufweist um selbsttragend seine Form beizubehalten und andererseits genügend Lumen bereitstellt, so dass eine Zellkultur erfolgen kann. Die Form der Außenoberfläche und der Innenoberfläche des Bodens kann dabei unabhängig voneinander entweder im Wesentlichen planar sein oder eine Krümmung aufweisen. Der Begriff "im Wesentlichen" bedeutet in diesem Zusammenhang, dass mindestens 75% der betreffenden Oberfläche die entsprechende Eigenschaft besitzen. Bevorzugt ist sowohl die Innenoberfläche als auch die Außenoberfläche im Wesentlichen planar ausgestaltet. An den Randbereichen, also den Bereichen an denen der Boden mit den Wänden des becherförmigen Einsatzes in Kontakt steht, kann auch ein Boden, der eine im Wesentlichen planare Innen- und/oder Außenoberfläche aufweist, eine Krümmung aufweisen.

Der becherförmige Einsatz kann unterschiedliche Grundformen aufweisen und kann, aber muss nicht, der Form der entsprechenden Vertiefung folgen, vorausgesetzt der becherförmige Einsatz ist im Wesentlichen passgenau einsetzbar. Beispielsweise kann die Öffnung, der Körper und/oder der Boden eines becherförmigen Einsatzes kreisförmig, ellipsoid, sternförmig oder anderweitig regelmäßig oder unregelmäßig, symmetrisch oder unsymmetrisch geformt sein.

Der becherförmige Einsatz kann eine Tiefe aufweisen, die einerseits durch die Tiefe der entsprechenden Vertiefung der zu verwendenden Multiwell-Platte begrenzt wird und andererseits für ein ausreichendes Lumen in dem becherförmigen Einsatz sorgt, so dass eine Zellkultur im Lumen möglich ist. Bevorzugt sind Tiefe und Ausgestaltung des Bodens des becherförmigen Einsatzes derart gewählt und auf die entsprechende Vertiefung abgestimmt, so dass nach erfolgtem Einsetzen des becherförmigen Einsatzes der Boden des Einsatzes planar auf dem Boden der Vertiefung zu liegen kommt. Bei dieser Ausgestaltung und unter der Voraussetzung, dass der becherförmige Einsatz und die verwendete Multiwell-Platte transparent ausgestaltet sind, ist eine optimale Mikroskopierbarkeit bzgl. der Innenoberfläche des Bodens des eingesetzten becherförmigen Einsatzes gegeben und sichergestellt. Im erfindungsgemäßen Verfahren ist bevorzugt mindestens ein Teil der becherförmigen Einsätze transparent ausgestaltet.

Eine Ausführung des becherförmigen Einsatzes ist auch in der Art möglich, dass die inneren Abmeddungen wie Durchmesser, Höhe und Volumen denen eines "wells" einer Multiwell-Platte mit der nächst höheren Wellzahl entspricht. Somit ist die zur Verfügung gestellte Zellkulturfläche und das Verhältnis von Oberfläche zu Volumen mit dem einer Vertiefung einer Multiwell-Platte mit der nächst höheren Wellzahl direkt vergleichbar.

Im erfindungsgemäßen Verfahren kann mindestens ein Teil der becherförmigen Einsätze transparent ausgestaltet sein.

Im erfindungsgemäßen Verfahren kann mindestens ein Teil der becherförmigen Einsätze derart dimensioniert sein, dass nach dem Einsetzen der becherförmigen Einsätze in jeweils eine Vertiefung einer Multiwell-Platte der Becherboden plan auf dem Boden der Vertiefung aufliegt.

Die becherförmigen Einsätze zur Verwendung im erfindungsgemäßen Verfahren können Mittel aufweisen, die eine Fixierung des becherförmigen Einsatzes in einer Vertiefung einer Multiwell-Platte gewährleisten. Unter einer Fixierung wird dabei verstanden, dass der Einsatz nach erfolgreichem Einsetzen in eine Vertiefung im Wesentlichen in seiner Position verbleibt und mindestens in einer Richtung nicht weiter bewegbar ist. Dazu kann der becherförmige Einsatz am oberen Becherrand Überhänge aufweisen, die nach dem Einsetzen des becherförmigen Einsatzes mit dem oberen Rand der Vertiefung in Kontakt stehen und somit ein weiteres Eindringen oder Absacken des Einsatzes in die Vertiefung verhindern.

Im erfindungsgemäßen Verfahren kann mindestens ein Teil der becherförmigen Einsätze Mittel aufweisen, die eine Fixierung des becherförmigen Einsatzes in einer Vertiefung einer Multiwell-Platte gewährleisten, bevorzugt weist mindestens ein Teil der becherförmigen Einsätze am oberen Becherrand Überhänge auf, die nach dem Einsetzen des becherförmigen Einsatzes mit dem oberen Rand der Vertiefung in Kontakt stehen.

Die becherförmigen Einsätze weisen mindestens auf der Innenseite des Bodens des Einsatzes jeweils einen ausgewählten Werkstoff und/oder eine ausgewählte Oberflächenstruktur auf. Bevorzugt werden becherförmige Einsätze verwendet, bei denen oder jeweils ausgewählte Werkstoff und/oder die jeweils ausgewählte Oberflächenstruktur auf der gesamten Innenoberfläche, bzw. der dem Lumen des Einsatzes zugewandten Oberfläche, des becherförmigen Einsatzes vorhanden ist. Es können aber auch solche becherförmigen Einsätze verwendet werden, bei denen nur der Becherboden den jeweils ausgewählten Werkstoff und/oder die jeweils ausgewählte Oberflächenstruktur aufweist oder daraus besteht. Besonders bevorzugt besteht der ganze becherförmige Einsatz aus dem ausgewählten zu testenden Werkstoff.

Im erfindungsgemäßen Verfahren kann mindestens ein Teil der becherförmigen Einsätze den jeweils ausgewählten Werkstoff und/oder die jeweils ausgewählte Oberflächenstruktur auf der gesamten Innenoberfläche des becherförmigen Einsatzes aufweisen.

Im erfindungsgemäßen Verfahren kann mindestens ein Teil der becherförmigen Einsätze derart ausgestaltet sein, dass nur der Becherboden den jeweils ausgewählten Werkstoff und/oder die jeweils ausgewählte Oberflächenstruktur aufweist oder daraus besteht.

Im erfindungsgemäßen Verfahren kann mindestens ein Teil der becherförmigen Einsätze aus dem jeweils ausgewählten Werkstoff bestehen.

Je nach Art und Beschaffenheit des zu testenden Werkstoffs können die becherförmigen Einsätze durch bekannte Verfahren hergestellt werden. Diese Verfahren umfassen Verfahren wie sie bei der Kunststoffformgebung Anwendung finden. Beispielsweise können Verfahren der Warmumformung angewendet werden, z.B. Spritzgießen, Spritzpressen, Pressen, Tiefziehen und/oder Schäumen. Es können auch Verfahren der Formgebung aus Lösungen zur Anwendung kommen, so z.B. Tauchen, Vakuumgießen und/oder Beschichten. Es sind auch andere Verfahren der Formgebung anwendbar, wie beispielsweise die spahnende Herstellung aus einem Voll- oder Massivwerkstoff. Es können auch verschiedene Herstellverfahren kombiniert werden, so z.B. Spritzprägen, Gasinnendruckverfahren, thermoplastischer Schaumguss, Mikroschaumverfahren oder sog. "insert moulding".

Sollte sich der zu testende Werkstoff nicht für die Herstellung eines kompletten becherförmigen Einsatzes eignen, so ist es ausreichend, wenn lediglich die dem Lumen zugewandte Innenoberfläche des Becherbodens das zu testende Material und/oder die zu testende Oberflächenstruktur aufweist. Dazu kann der zu testende Werkstoff einem becherförmigen Einsatz beispielsweise mittels hinterspritzen zugeführt werden.

Grundsätzlich ist die Art der Herstellung des becherförmigen Einsatzes für das erfindungsgemäße Verfahren unerheblich. Bevorzugt werden Verfahren eingesetzt, die die Herstellung der becherförmigen Einsätze ohne den Einsatz von Lösungsmitteln erlauben. Solche Verfahren umfassen beispielsweise Verfahren zur Warmumformung bzw. thermoplastischen Verarbeitung wie z.B. Spritzgießen, Spritzpressen, Pressen und Tiefziehen.

Im erfindungsgemäßen Verfahren kann mindestens ein Teil eines becherförmigen Einsatzes mittels eines Hinterspritzungsverfahrens hergestellt sein.

Im erfindungsgemäßen Verfahren kann, z.B. nach erfolgter Herstellung des Einsatzes, der becherförmige Einsatz einer Oberflächenmodifikation unterzogen werden, um ggf. zu testende Oberflächenstrukturen bereitzustellen. Diese Oberflächenmodifikationen können durch chemische Reaktionen hervorgerufene Modifikationen umfassen, wie beispielsweise die Einführung von funktionalen Gruppen, die mindestens eine Eigenschaft der behandelten Oberfläche verändern, sowie Plasmabehandlungen und/oder andere Verfahren zur physikalischen Oberflächenmodifikation. Physikalische Verfahren umfassen dabei solche Verfahren bei denen mindestens eine luminale Oberfläche des Einsatzes mit einer Komponente behandelt wird, die für den verwendeten Werkstoff des Einsatzes als Lösungsmittel wirkt, gefolgt von der Zugabe einer Komponente, die für die entstandene Lösung ein Fällmittel darstellt. Eine solche Behandlung bewirkt nicht nur eine Veränderung der Oberflächenmorphologie am Becher, sondern kann ggf. zusätzlich Möglichkeiten für weitere Oberflächenmodifikationen eröffnen, beispielsweise durch anschließende chemische Modifikationen. Es sind auch Verfahren umfasst, bei denen z.B. durch Verschäumungsprozesse mit Gasen im überkritischen Zustand, bevorzugt von überkritischem CO₂, verwendet werden um Oberflächenmodifikationen hervorzurufen. Auch können bereits durch das verwendete Herstellverfahren für die becherförmigen Einsätze selbst Oberflächenstrukturierungen hervorgerufen werden, z.B. beim Hinterspritzen. Es können auch Laserverfahren oder andere Verfahren zum gezielten Materialabtrag eingesetzt werden. Schließlich sind auch Kombinationen von verschiedenen Verfahren zur Oberflächemodifikation umfasst.

Durch solche Oberflächenmodifikationen können becherförmige Einsätze mit Oberflächen mit variierenden Parametern beispielsweise hinsichtlich der Werkstoffzusammensetzung an der Oberfläche, des Oberflächenpotentials, der Porosität, der Mikro- und/oder Makrostrukturierung, der Hydrophobizität, der Hydrophilizität und/oder der Ausstattung mit funktionellen Gruppen bereitgestellt und einer Testung zugeführt werden.

Im erfindungsgemäßen Verfahren kann mindestens ein becherförmiger Einsatz auf der Becherinnenseite eine Oberflächenstruktur aufweisen, die erst nach erfolgter Formung des becherförmigen Einsatzes erzeugt worden ist.

Im erfindungsgemäßen Verfahren werden die becherförmigen Einsätze jeweils in eine Vertiefung von Multiwell-Platten entsprechender Dimensionierung eingesetzt, so dass die Außenseite des Becherbodens in Richtung der Innenseite des Bodens der jeweiligen Vertiefung der Multiwell-Platte zeigt. Dadurch ist sichergestellt, dass das Lumen der becherförmigen Einsätze nach erfolgreichem Einsetzen über die Öffnung von außen zugänglich bleibt, so dass die Bestandteile der Zellkultur zugeführt werden können.

In einem weiteren Schritt des erfindungsgemäßen Verfahrens erfolgt nun eine Kultur von Zellen im Lumen der in die passenden Vertiefungen eingesetzten becherförmigen Einsätze. Die Bedingungen für die Zellkultur sowie die Parameter für die Bewertung der erzielten Testergebnisse hängen von der jeweiligen Fragestellung ab. Das erfindungsgemäße Verfahren ist nicht auf die Kultur bestimmter Zellen oder die Testung bestimmter Materialien oder ausgewählter Bedingungen beschränkt sondern eignet sich für eine Vielzahl von Anwendungen.

Es wird auch ein Kit zur Durchführung eines erfindungsgemäßen Verfahrens offenbart, wobei der Kit dadurch gekennzeichnet ist, dass der Kit eine Mehrzahl an verschiedenen, oben näher beschriebenen, becherförmigen Einsätzen umfasst. Bevorzugt ist die Anzahl der becherförmigen Einsätze eines Kits teilbar durch die Anzahl der Vertiefungen einer Multiwell-Platte auf deren Dimensionierung die becherförmigen Einsätze abgestimmt sind. Des Weiteren kann der Kit eine Beschreibung der Werkstoffe und/oder Oberflächenstrukturen umfassen, die von den becherförmigen Einsätzen des Kits bereitgestellt werden.

### Figuren:

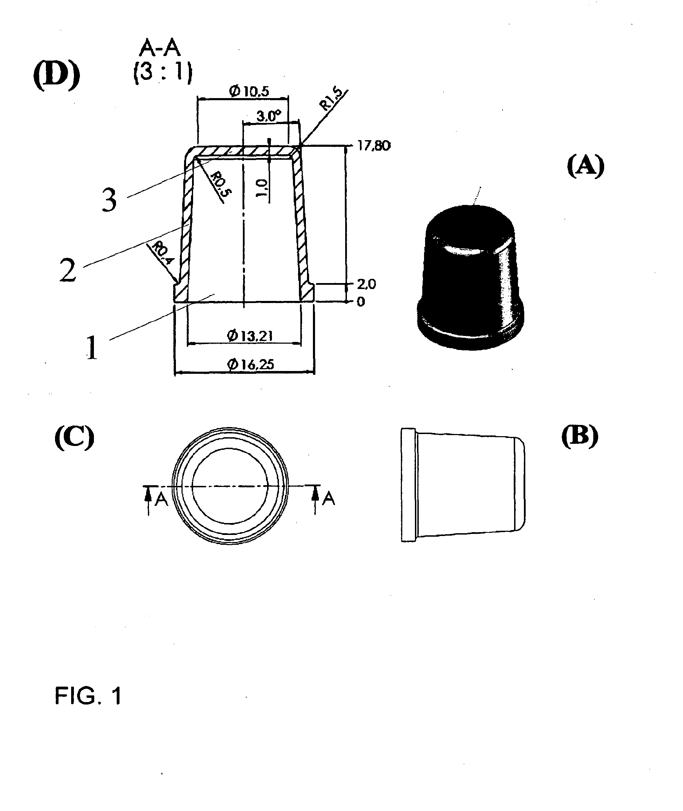

- FIG. 1: zeigt eine Ausführungsform eines becherförmigen Einsatzes zur Verwendung mit einer 24-well Multiwell-Platte des Herstellers Corning Inc., USA, wobei in (A) eine perspektivische Draufsicht dargestellt ist, in (B) eine Seitenansicht, in (C) eine Aufsicht mit Angabe einer Schnittebene A-A und in (D) eine schematische Schnittansicht entlang der Schnittebene A-A.

Im Folgenden wird die Erfindung anhand von Ausführungsbeispielen näher erläutert.

### Beispiele:

Ausführung eines becherförmigen Einsatzes für eine 24-well Multiwell-Platte des Herstellers Corning Inc., USA:

Der in Fig. 1 (A) bis (D) schematisch dargestellte, becherförmige Einsatz weist eine Öffnung 1, eine umlaufende Wand 2 und einen Boden 3 auf. Dabei weist der Einsatz eine Grundform in der Form eines konischen Kegelstumpfes auf, wobei die Grundfläche der Öffnung 1 größer ist als die Grundfläche des Bodens 3. Am dem Boden 3 abgewandten Ende des Einsatzes weist der Einsatz einen Überhang auf, der der Fixierung des Einsatzes in der Vertiefung dient. Der Boden 3 ist im Wesentlichen planar ausgestaltet und ist auf seiner Außenoberfläche poliert. Auf der dem Lumen zugewandten Innenoberfläche weist der Boden 3 ein zu testender Werkstoff auf. Die Tiefe des Einsatzes ist derart gewählt, dass nach erfolgreichem Einsetzen des Bechers in eine Vertiefung einer 24-well Multiwell-Platte der Firma Corning Inc. der Boden 3 des Einsatzes planar auf dem Boden der Vertiefung aufliegt. Sind sowohl der Einsatz als auch die Multiwell-Platte transparent ausgestaltet, so können Zellen, die auf der Innenoberfläche des Bodens des Einsatzes wachsen, mit herkömmlichen, in der Zellpräparation üblichen Mikroskopen (z.B. invers) mikroskopiert werden.

## Patentansprüche

1. Verfahren zur Testung verschiedener, ausgewählter Werkstoffe und/oder Oberflächenstrukturen für die Kultur von Zellen und/oder Mikroorganismen, umfassend die Schritte:
i) Bereitstellung einer Mehrzahl von zu testenden verschiedenen, ausgewählten Werkstoffen und/oder Oberflächenstrukturen in Form von becherförmigen Einsätzen, wobei die becherförmigen Einsätze derart dimensioniert sind, dass ein becherförmiger Einsatz jeweils in eine Vertiefung einer für die Zellkultur geeigneten Multiwell-Platte im Wesentlichen passgenau einsetzbar ist, wobei jeder becherförmige Einsatz Wände, einen Boden und auf der dem Boden abgewandten Seite eine Öffnung aufweist, wobei Wände und Boden der becherförmigen Einsätze flüssigkeitsdicht ausgestaltet sind und wobei jeder becherförmige Einsatz mindestens auf der Bodeninnenseite jeweils einen ausgewählten Werkstoff und/oder eine ausgewählte Oberflächenstruktur aufweist;
ii) Einsetzen der becherförmigen Einsätze in Vertiefungen von Multiwell-Platten entsprechender Dimensionierung, so dass die Außenseite des Becherbodens in Richtung der Innenseite des Bodens einer Vertiefung der Multiwell-Platte zeigt;
iii) Kultur der Zellen im Lumen der becherförmigen Einsätze.

2. Verfahren nach Anspruch 1, **dadurch gekennzeichnet, dass** mindestens ein Teil der becherförmigen Einsätze derart dimensioniert ist, dass ein becherförmiger Einsatz im Wesentlichen passgenau in eine Vertiefung einer Multiwell-Platte im 4-well, 6-well, 12-well, 24-well, 48-well, 96-well oder 384-well Format einsetzbar ist, bevorzugt in eine Vertiefung einer 24-well Multiwell-Platte.

3. Verfahren nach einem der vorhergehenden Ansprüche, **dadurch gekennzeichnet, dass** mindestens ein Teil der becherförmigen Einsätze Mittel aufweist, die eine Fixierung des becherförmigen Einsatzes in einer Vertiefung einer Multiwell-Platte gewährleisten, bevorzugt weist mindestens ein Teil der becherförmigen Einsätze am oberen Becherrand Überhänge auf, die nach dem Einsetzen des becherförmigen Einsatzes mit dem oberen Rand der Vertiefung in Kontakt stehen.

4. Verfahren nach einem der vorhergehenden Ansprüche, **dadurch gekennzeichnet, dass** mindestens ein Teil der becherförmigen Einsätze transparent ausgestaltet ist.

5. Verfahren nach einem der vorhergehenden Ansprüche, **dadurch gekennzeichnet, dass** mindestens ein Teil der becherförmigen Einsätze derart dimensioniert ist, dass nach dem Einsetzen des becherförmigen Einsatzes in eine Vertiefung einer Multiwell-Platte der Becherboden plan auf dem Boden der Vertiefung aufliegt.

6. Verfahren nach einem der vorhergehenden Ansprüche, **dadurch gekennzeichnet, dass** mindestens ein Teil der becherförmigen Einsätze der jeweils ausgewählte Werkstoff und/oder die jeweils ausgewählte Oberflächenstruktur auf der gesamten Innenoberfläche des becherförmigen Einsatzes aufweist.

7. Verfahren nach einem der vorhergehenden Ansprüche, **dadurch gekennzeichnet, dass** mindestens ein Teil der becherförmigen Einsätze aus dem jeweils ausgewählten Material bestehen.

8. Verfahren nach einem der Ansprüche 1 bis 5, **dadurch gekennzeichnet, dass** mindestens ein Teil der becherförmigen Einsätze derart ausgestaltet sind, dass nur der Becherboden den jeweils ausgewählten Werkstoff und/oder die jeweils ausgewählte Oberflächenstruktur aufweist oder daraus besteht.

9. Verfahren nach Anspruch 8, **dadurch gekennzeichnet, dass** mindestens ein Teil des becherförmigen Einsatzes mittels eines Hinterspritzungsverfahrens hergestellt ist.

10. Verfahren nach einem der vorhergehenden Ansprüche, **dadurch gekennzeichnet, dass** mindestens ein becherförmiger Einsatz auf der Becherinnenseite eine Oberflächenstruktur aufweist, die erst nach erfolgter Formung des becherförmigen Einsatzes erzeugt worden ist.

## Claims

1. A process for testing different, selected materials and/or surface structures for the culture of cells and/or micro-organisms, comprising the steps of:
i) providing a plurality of different, selected materials and/or surface structures to be tested in the form of cup-shaped inserts, which cup-shaped inserts are dimensioned such that each cup-shaped insert can be inserted, and substantially fits, in a depression of a multi-well plate suitable for cell culture, wherein each cup-shaped insert comprises walls, a bottom, and an opening on the side facing away from the bottom, wherein the walls and the bottom of the cup-shaped inserts are liquid-tight, and wherein each cup-shaped insert comprises a selected material and/or a selected surface structure at least on the inner side of the bottom;
ii) inserting the cup-shaped inserts in depressions of multi-well plates of suitable dimensions, so that the outer side of the cup's bottom faces towards the inner side of the bottom of a depression of the multi-well plate;
iii) culture of the cells within the lumen of the cup-shaped inserts.

2. The process according to claim 1, **characterized in that** at least part of the cup-shaped inserts are dimensioned such that a cup-shaped insert can be inserted, and substantially fits, in a depression of a multi-well plate having a 4-well, 6-well, 12-well, 24-well, 48-well, 96-well, or 384-well format, preferably in a depression of a multi-well plate having 24 wells.

3. The process according to any one of the preceding claims, **characterized in that** at least part of the cup-shaped inserts comprise means which ensure that the cup-shaped insert is fixed in position in a depression of a multi-well plate, wherein at least part of the cup-shaped inserts preferably comprise projections on the upper edge of the cup, which are in contact with the upper edge of the depression once the cup-shaped insert has been inserted.

4. The process according to any one of the preceding claims, **characterized in that** at least part of the cup-shaped inserts are transparent.

5. The process according to any one of the preceding claims, **characterized in that** at least part of the cup-shaped inserts are dimensioned such that the cup's bottom rests flat on the bottom of the depression once the cup-shaped insert has been inserted in a depression of a multi-well plate.

6. The process according to any one of the preceding claims, **characterized in that** at least part of the cup-shaped inserts comprise the selected material and/or the selected surface structure on the entire inner surface of the cup-shaped insert.

7. The process according to any one of the preceding claims, **characterized in that** at least part of the cup-shaped inserts consist of the selected material.

8. The process according to any one of claims 1 to 5, **characterized in that** at least part of the cup-shaped inserts are designed such that only the cup's bottom comprises, or consists of, the selected material and/or the selected surface structure.

9. The process according to claim 8, **characterized in that** at least a part of the cup-shaped insert is made by means of a back injection process.

10. The process according to any one of the preceding claims, **characterized in that** at least one cup-shaped insert comprises, on the inner side of the cup, a surface structure which has been produced after the cup-shaped insert has been formed.

## Revendications

1. Procédé pour examiner différents matériaux et/ou structures extérieures sélectionnés pour la culture de cellules et/ou de microorganismes, comprenant les étapes suivantes :
i) fourniture d'une multiplicité de différents matériaux et/ou structures extérieures sélectionnés à examiner sous forme de charges en forme de coupelle, les charges en forme de coupelle étant dimensionnées de telle sorte qu'une charge en forme de coupelle peut être respectivement introduite de façon essentiellement ajustée dans un creux d'une platine Multiwell appropriée pour la culture cellulaire, chaque charge en forme de coupelle présentant des parois, un fond et une ouverture sur le côté opposé au fond, des parois et un fond des charges en forme de coupelle étant constitués de façon étanche aux liquides, et chaque charge en forme de coupelle présentant au moins sur le côté intérieur du fond respectivement un matériau sélectionné et/ou une structure extérieure sélectionnée ;
ii) introduction des charges en forme de coupelle dans des creux de platines Multiwell d'un dimensionnement correspondant de telle sorte que le côté extérieur du fond de coupelle est dirigé en direction du côté intérieur du fond d'un creux de la platine Multiwell ;
iii) culture des cellules dans la lumière des charges en forme de coupelle.

2. Procédé selon la revendication 1, **caractérisé en ce qu'**au moins une partie des charges en forme de coupelle est dimensionnée de telle sorte qu'une charge en forme de coupelle peut être introduire de façon essentiellement ajustée dans un creux d'une platine Multiwell au format 4-well, 6-well, 12-well, 24-well, 48-well, 96-well ou 384-well, de préférence dans un creux d'une platine Multiwell 24-well.

3. Procédé selon une des revendications précédentes, **caractérisé en ce qu'**au moins une partie des charges en forme de coupelle présente des moyens qui garantissent une fixation de la charge en forme de coupelle dans un creux d'une platine Multiwell, de préférence **en ce qu'**au moins une partie des charges en forme de coupelle présente, sur le bord de coupelle supérieur, des saillies qui, après l'introduction de la charge en forme de coupelle, sont en contact avec le bord supérieur du creux.

4. Procédé selon une des revendications précédentes, **caractérisé en ce qu'**au moins une partie des charges en forme de coupelle est constituée de façon transparente.

5. Procédé selon une des revendications précédentes, **caractérisé en ce qu'**au moins une partie des charges en forme de coupelle est dimensionnée de telle sorte que, après l'introduction de la charge en forme de coupelle dans un creux d'une platine Multiwell, le fond de coupelle repose à plat sur le fond du creux.

6. Procédé selon une des revendications précédentes, **caractérisé en ce qu'**au moins une partie des charges en forme de coupelle présente le matériau respectivement sélectionné et/ou la structure extérieure respectivement sélectionnée sur la totalité de la surface intérieure de la charge en forme de coupelle.

7. Procédé selon une des revendications précédentes, **caractérisé en ce qu'**au moins une partie des charges en forme de coupelle se compose du matériau respectivement sélectionné.

8. Procédé selon une des revendications 1 à 5, **caractérisé en ce qu'**au moins une partie des charges en forme de coupelle est constituée de telle sorte que seul le fond de coupelle présente le matériau respectivement sélectionné et/ou la structure extérieure respectivement sélectionnée ou en est composé.

9. Procédé selon la revendication 8, **caractérisé en ce qu'**au moins une partie de la charge en forme de coupelle est fabriquée au moyen d'un procédé de surmoulage par injection.

10. Procédé selon une des revendications précédentes, **caractérisé en ce qu'**au moins une charge en forme de coupelle présente, sur le côté intérieur de coupelle, une structure extérieure qui n'a été produite qu'à la suite de la réalisation de la formation de la charge en forme de coupelle.
